# EUROPEAN PATENT SPECIFICATION

(11) **EP 1 284 780 B1**
(45) Date of publication and mention of the grant of the patent: **07.05.2008**
(21) Application number: 01937293.7
(22) Date of filing: 10.05.2001
(51) Int. Cl.: A61M 5/32, A61L 29/14

(54) **A CATHETER LOCK SOLUTION INCLUDING A PHOTO-OXIDANT**
KATHETERSPERRLÖSUNG MIT PHOTO-OXIDANT
SOLUTION DE CONTROLE DE SONDE CONTENANT UN PHOTO-OXYDANT

(30) Priority: 10.05.2000 US 203358 P
(43) Date of publication of application: 26.02.2003
(73) Proprietor: Ash Access Technology, Inc., Lafayette, Indiana 47904 (US)
(72) Inventor: ASH, Stephen, R., Lafayette, IN 47905 (US)
(74) Representative: Atkinson, Peter Birch
(86) International application number: PCT/US2001/015177
(87) International publication number: WO 2001/085249

(56) References cited:
- WO-A1-00/10385
- US-A- 5 002 965
- US-B1- 6 207 107

## Description

### BACKGROUND OF THE INVENTION

This invention generally relates to catheters and methods of preventing infection of catheters, such as intravascular catheters and other body cavity catheters. More specifically, but not exclusively, this invention relates to infusing a lock solution into an indwelling catheter, such as, for example, an indwelling intravascular catheter, for inhibiting infection in an animal having an indwelling catheter.

By way of background, catheters are used with increasing frequency to treat patients requiring a variety of medical procedures. The catheters offer many advantages for patients; for example, catheters provide ready access without repeated injections for administration of large volumes of fluids, nutrients, medications and withdrawal of blood. For example, catheters can be used for infusion of fluids such as drugs, electrolytes or fluids used in chemotherapy, or for the removal of blood on an intermittent basis. For example, in hyperalimentation treatment, the catheters are usually used for infusion of large volumes of fluids. In chemotherapy, catheters are used for infusion of drugs on an intermittent basis, ranging from daily to weekly. For hemodialysis, dual-lumen catheters are typically used--usually three times per week; one lumen allows removal of blood, while the other lumen allows blood to return.

Catheters can either be acute or temporary for short-term use or chronic for long-term treatment. Catheters are commonly inserted into central veins (such as the vena cava) from peripheral vein sites. Another alternative is placement of a dual-lumen chronic (tunneled and cuffed) CVDC through the internal jugular vein. Adequate hemodialysis requires removal and return of 250-400 mL of blood per minute. Great care must be taken in the placement and use of a chronic catheter to prevent infection of the patient at the site of access or within the vascular system.

Chronic venous catheters usually include a DACRON cuff attached to the catheter and placed under the skin, which promotes ingrowth of fibrous tissue, fixes the catheter in position, and prevents bacterial migration around the catheter. Most chronic central venous dialysis catheters ("CVDCs") in use in the U.S. today have single subcutaneous Dacron^{®} cuffs, placed in the tunnel, 1-4 cm beneath the skin exit site. For dual lumen catheters such as the Ash Split Cath^{™} and Bard Hickman^{®} catheters, there is one cuff on the catheter. For single-lumen catheters such as Tesio^{®} catheters, there is a single Dacron cuff for each catheter. For these cuffed, tunneled CVDC there is no apparent or demonstrated difference in the rate of exit site infection or catheter-related bloodstream infection ("CRBSI"). It is believed that the only chronic CVDC in the U.S. at present that does not have a subcutaneous Dacron cuff is the Schoen^{™} catheter. In this catheter a subcutaneous plastic clip connects two Tesio catheters. This clip fixes the catheters in position and apparently prevents pericatheter bacterial migration in a manner similar to a Dacron cuff. Chronic CVDCs are typically made from one of three types of materials: silicone, polyurethane, or polyurethane derivatives.

Catheters, especially chronic venous catheters, have drawbacks. The use of both temporary and chronic CVDC is associated with certain complications that may require catheter removal, catheter replacement or administration of medical therapies. They can became occluded by a thrombus, and even if extreme care is taken, the catheters can increase a patent's risk of infection. Intraluminal thrombus formation may impair catheter flow, as can thrombus formation just outside the tip of the catheter. Impairment of the flow may lead to catheter removal or administration of drugs such as tPA to resolve these thromboses.

In order to prevent clotting of catheters in blood vessels between uses, the catheters are usually filled with a lock solution that comprises a concentrated solution of the commonly used anticoagulant, heparin (usually up to 10,000 units of heparin per catheter lumen). The heparin lock solution is injected into each lumen immediately after each use, and typically left in the catheter until the catheter is accessed again. The heparin lock solution is then withdrawn from the catheter before the next use because infusing this amount of heparin into a patient's bloodstream runs the risk of causing excessive bleeding. During the catheter lock procedure the injected volume of solution is preferably exactly the same as the internal volume of the catheter.

Even when this volume is injected exactly, about 1/3 of the injected anticoagulant volume typically leaves the end of the catheter, causing some anticoagulation of the patient in the hours after a dialysis procedure.

In addition, even with the use of a heparin lock solution, the catheter can become occluded between uses from coagulation of blood in the catheter. Blood may be found in the catheter because, for example, an inadequate volume of heparin was infused within the catheter lumen, the heparin diffused from the lumen, or residual blood remains in the lumen during the catheter lock. This often results in formation of a thrombus with concomitant loss of flow through the lumen. The occluded catheters frequently are removed and/or replaced.

Furthermore, it has been reported that thrombi and fibrin deposits on catheters may serve as a nidus for microbial colonization of the intravascular devices, and that catheter thrombosis might be one factor associated with infection of long-term catheters. Thus the use of anticoagulants (e.g., heparin or citrate) or thrombolytic agents may have a role in the prevention of catheter-related bloodstream infections. However, recent in vitro studies suggest that the growth of coagulase-negative Staphylococci on catheters may also be enhanced in the presence of heparin. In some patients the routine use of heparin to maintain catheter patency, even at doses as low as 250 to 500 units per day, has caused heparin induced thrombocytopenia (HIT Syndrome) in patients with anti-heparin antibodies. This serious syndrome can result in severe and sudden thromboembolic and hemorrhagic complications.

Heparin solutions have no proven intrinsic antiseptic properties to prevent infection after catheter hub contamination. "Antiseptic", as used herein, means "relating to the prevention of infection by inhibiting the growth of infectious agents", as defined in Stedman's medical dictionary. Heparin, in fact, may help to promote growth of bacteria within the "biofilm" layer of protein on the catheter surfaces (protamine has the opposite effect). The "biofilm" proteins on the catheter surfaces can protect bacteria from antibiotics and white cells. Also, heparin induces the loss of platelets and, paradoxically, can induce clotting in some patients (the "white clot" syndrome). In addition, catheters, particularly venous catheters, are frequently accessed with syringes, or uncapped and directly connected to IV lines, creating a situation wherein the probability of microbial infection is relatively high.

Studies have shown that catheter-related bloodstream infection ("CRBSI") in hemodialysis patients is caused most frequently by Staphylococcus species such as S Epidermidis. However, hemodialysis patients are reported to have a greater proportion of CRBSIs due to S. Aureus than do other patient populations and a significant number of infections are due to gram-negative organisms. The lack of antiseptic properties of a 5000 U/mL heparin lock was confirmed by a study performed by BEC Laboratories, Inc. under the standard USP antimicrobial effectiveness test protocol.

Significant resources are currently being invested in a search for alternatives to heparin for catheter lock that do not have the above disadvantages. One alternative developed by the present inventor is to use concentrated sodium citrate, as described in International Publication No. WO 00/10385. Concentrated citrate is a more effective anticoagulant than heparin, especially in patients with deficiency of antithrombin-III (such as patients with liver failure). In addition, citrate will not cause peripheral anticoagulation of the patient if some of the injected citrate enters the circulation, since it is rapidly metabolized and distributed. Furthermore, it was discovered that concentrated citrate had additional advantages when used in a lock solution.

Nevertheless, if a great excess of heparin or citrate is injected into the patient's blood during a catheter lock procedure or by mistaking heparin or citrate as some other fluid (such as saline) that is injected during the dialysis procedure, then harm can come to the patient. For citrate, rapid injections of large volumes of sodium citrate solution can cause transient symptoms of hypocalcemia, hypotension and arrhythmia. A transient decrease in calcium level can cause cardiac arrhythmia. Excess heparin can cause excess anticoagulation of the patient's blood and bleeding from a number of sites.

A significant problem is that all of the fluids used in a dialysis unit, including heparin, citrate, saline, and lidocaine (anesthetic) are all clear. Once a syringe is filled with a fluid, it is difficult or impossible to tell what is the fluid within the syringe absent careful marking of the syringe. Though attempts are made to label syringes after filling them when they are pre-filled, it is up to the user to remember what fluids they have just drawn into syringes.

As further background, another complication associated with chronic CVDC is infection. As noted above, when catheters are inserted into veins or arteries, they bypass the protective dermis layer, and provide direct access to a patient's blood stream. The same applies for insertion of a catheter into another body cavity. This can cause the inadvertent transfer of infectious agents into the vein or artery at the location of the catheter.

Hemodialysis catheters may become contaminated by a variety of mechanisms. During placement of the catheter and early use if there is bacterial contamination of the catheter, bloodstream infection is seen several days to weeks later. Later in the use of catheters other factors determine the risk of infection, including, for example, the following: (a) penetration of organisms around the catheter from the skin following exit site infection; (b) contamination of catheter connections during attachment of dialysis tubing or syringes; (c) contamination of blood as it passes through the dialysis system, (d) administration of contaminated blood or other solutions through the catheter during or after the dialysis session or, (e) endogenous bloodstream infection during or between dialysis treatments.

For the above reasons, catheters have a propensity to become contaminated. Bloodstream infection and localized infections of the skin exit site are common in hemodialysis patients. If there is bacteremia (bacteria in blood), then the catheter surfaces within the vein or artery can become seeded with bacteria. In either case, the patient can develop septicemia (infection in the blood) and become seriously ill.

For chronic CVDC the most common cause of catheter infection is contamination of the connector hub. The predominant route of contamination is endoluminal. The major determinant of the rate of infection is the frequency with which the catheter hub is opened and the major preventive step is the care in disinfection of the hub and prevention of contamination of the hub. Since endoluminal contamination is the major cause of CRBSI in chronic CVDC, the determinants of infection center on the procedures and handling of the catheter.

In addition, the foreign surfaces of catheters can create a smooth surface at which bacteria can grow, and at which the white cells are unable to surround or "phagocytize" the bacteria. Several studies have indicated a rate of bloodstream infection during use of chronic CVDC of 1.1 per 1,000 patient days to 2.2 per 1,000 patient days. One study demonstrated a catheter-related bacteremia rate of 2.2 to 3.8 bacteremic episodes per 1,000 patient days, the lower rate being for catheters placed surgically rather than radiologically. Another study of new tunneled catheters reported that 19% of catheters became infected in a mean of 62 days after catheter placement, representing a rate of 3 infections per 1,000 days. This means that each patient has approximately a 10% chance of developing bloodstream infection during each month. There is no evidence that the rate of CRBSI increases with duration of use of a chronic CVDC. In fact, practical experience and various studies have shown that the rate of CRBSI is the same over the many months of use. Tests indicate that the risk of CRBSI is the same for each period of time that the patient has a catheter. Over time the patient has a higher chance for infection merely because there is more time at risk for infection. The longer the patients have chronic CVDC, the greater the chance that an infection will occur, but this is merely due to greater time for a constant risk of exposure.

CRBSI in dialysis patients is usually associated with modest symptoms and clears after antibiotic therapy. However, in some patients, signs of infection are much more severe and include all of the symptoms of Systemic Inflammatory Response Syndrome ("SIRS") (tachycardia, tachypnea, abnormal temperature and white count) plus hypotension. Often these patients must be hospitalized and given intravenous antibiotics. In spite of this care, patients often remain seriously ill until the infected catheter is removed.

Similarly SIRS can occur in ICU patients with CRBSI due to central venous catheters. The mortality rate following CRBSI in ICU patients has been reported to be 3-25%. Of the 300,000 patients on dialysis in the U.S., about 60,000 have chronic CVDC. Assuming an average incidence of CRBSI of only 21,000 patient-days at risk, about 120 of these patients develop CRBSI each day. At the lowest reported mortality rate of 3%, 3-4 ESRD patients die from CRBSI each day. At the highest reported mortality of 25%, 30 ESRD patients die from CRBSI each day. The cost attributable to caring for a single CRBSI episode in hospitalized patients has been reported to be between $3,700 and $29,000. Costs may be similar for patients with CRBSI related to chronic CVDC, given the higher cost of removing and replacing a chronic CVDC. Given the serious consequences of CRBSI, the acute illness of the patient who apparently has bacteremia, and the frequent decision to remove the catheter on the presumption that it is the source, there is a great need for alternative means for fighting catheter infection.

Furthermore, because of frequent hospitalizations and receipt of antibiotics to treat bloodstream and vascular access infections, hemodialysis patients are at high risk for infection with drug-resistant bacteria. Studies have shown, that among vascular access types, arteriovenous fistulas created from the patient's own blood vessels have the lowest rates of infection; grafts constructed from synthetic materials have intermediate risk; and central catheters have the highest risk. The rapid increase in vancomycin-resistant enterococci (VRE) in the United States has been attributed to use of antimicrobials, especially empirically prescribed vancomycin. Vancomycin is used commonly in dialysis patients for empiric therapy of symptoms of bloodstream infection because it can be administered once a week and is effective against two common pathogens, coagulase-negative Staphylococci and Staphylococcus Aureus. The greater the use of vancomycin, however, the greater the risk of inducing vancomycin-resistant staphylococcus, and if this is the cause of septicemia, there are then no effective drugs with which to treat these patients. Use of prophylactic vancomycin and other antibiotics to prevent catheter infection is therefore discouraged, and alternate means for fighting catheter infection are greatly needed.

Catheters are also used to convey fluids into and out of other body cavities besides veins, as noted above. Catheters are placed into arteries to measure blood pressure or remove arterial blood for analysis of gases reflecting lung function. Catheters are placed into the peritoneum (the space surrounded by the peritoneal membrane and external to organs in the abdomen) to perform peritoneal dialysis and remove fluids and toxins from the patient. Other catheters are placed into the fluid around the nervous system (cerebral spinal fluid) for removal of this fluid or administration drugs, and into the subcutaneous space for administration of various drugs or fluids. Such catheters are also subject to infection and to other problems addressed herein.

Thus in light of the above described problems, there is a continuing need for advancements in the field of catheter lock solutions. The present invention is such an advancement and provides a wide variety of benefits and advantages.

### SUMMARY OF THE INVENTION

In one aspect, the present invention provides a catheter lock solution (also referred to herein as a fluid) that includes citrate as an anticoagulant and a photo-oxidant that has an antiseptic effect. In one embodiment, the photo-oxidant is methylene blue, and in other embodiments other photo-oxidants are used. The solution in other embodiments also includes a viscosifying agent and/or additional pharmaceutically acceptable materials. In certain embodiments, the pH is controlled to enhance the safety and the effectiveness of the solution. The relative density of the solution is also selected in certain embodiments to optimize the length of time that the solution remains in a catheter.

The fluid may have a pH of from about 6 to about 8, more preferably about 7.0 to about 7.4.

The solution of the invention is particularly useful in treating a patient having an infection or a substantial risk of infection related to the presence of the catheter. When a solution including methylene blue is used, the solution can also be exposed the to light or other radiant energy to enhance the antiseptic properties of the solution.

A second aspect of the invention the use of an anticoagulant and a photo-oxidant, wherein the photo-oxidant has an antiseptic effect, for the manufacture of a medicament in the form of a catheter lock solution for infusion into the lumen of a catheter for preventing passage of infection along the catheter.

A third aspect of the invention provides an infusion device for infusing a lock solution into a lumen for a catheter, said device comprising:
a syringe;
a pharmaceutically acceptable lock solution contained within the syringe, said lock solution including citrate as an anticoagulant and a photo-oxidant wherein the photo-oxidant has an antiseptic effect and said syringe containing the lock solution is sterilized.

A fourth aspect of the invention provides a kit for locking a patient's cathether, comprising:
a container having therein a catheter lock solution, the catheter lock solution comprising citrate as an anticoagulant and a photo-oxidant, wherein the photo-oxidant has an antiseptic effect;
a syringe with Luer lock tip;
a replacement cap; and
a needleless single-dose via access spike with dead cap.

Further objects, features, aspects, forms, advantages and benefits shall become apparent from the description and drawings contained herein.

While the actual nature of the invention covered herein can only be determined with reference to the claims appended hereto, certain forms and features, which are characteristic of the preferred embodiments disclosed herein, are described briefly as follows:

### BRIEF DESCRIPTION OF THE DRAWINGS

Figure 1 is a perspective view of one embodiment of a catheter and syringe for infusing a lock solution into a catheter for use with the present invention.
Figure 2 is a graph depicting the effect of methylene blue (12 mg/100ml) on concentration of a gram negative organism, Escherichia Coli, without light exposure. All E. Coli are killed within one day.
Figure 3 is a representation of the effect of methylene blue (12 mg/100ml) on concentration of a gram positive organism, Enterobacter Faecalis, without light exposure. All Enterobacter are killed within one day.
Figure 4 is a representation of antibacterial properties of methylene blue 0.01% in ACD (4% citrate) for gram positive organisms and gram negative organisms.
Figure 5 is a representation of antiseptic properties of methylene blue 0.01% in 0.24 Molar citrate buffer at pH 4.5 and 7.2 for gram-positive organisms and gram-negative organisms.
Figure 6 depicts a linear graph showing the relationship of densities of various catheter lock solutions to citrate concentration.

### DETAILED DESCRIPTION OF THE INVENTION

For the purposes of promoting an understanding of the principles of the invention, reference will now be made to the embodiments illustrated herein and specific language will be used to describe the same. It will nevertheless be understood that no limitation of the scope of the invention is thereby intended. Any alterations and further modifications in the described processes, systems or devices, and any further applications of the principles of the invention as described herein, are contemplated as would normally occur to one skilled in the art to which the invention relates.

In accordance with the invention a catheter lock solution is used to provide anticoagulant and antibacterial properties to an implanted catheter as the lock solution resides in the catheter between uses. As used herein, the term "lock solution" refers to a solution that is injected or otherwise infused into a lumen of a catheter with the intention of allowing at least a portion of a lock solution to remain in the lumen until it is desired or required to access that particular lumen again, typically for additional treatment, i.e., infusion or withdrawal of fluid. It is desired that at least a portion the lock solution remain in the lumen for a desired amount of time lasting from about 1 hour to 3 or 4 days or longer. However, frequently the lock solution is changed on a daily basis during regular care and sterile maintenance of the indwelling catheter. Use of a lock solution in accordance with the present invention provides particular advantages for patients with catheters by inhibiting catheter-related infections and by preventing catheter occlusion.

Catheters used in connection with the present invention typically can either be acute (temporary) or chronic (long-term) catheters surgically implanted in the animal. The catheters usually are inserted into a vein or artery. The catheters are typically used in varying intervals to administer fluids, nutrients, and medications into the body. The catheters also can be used to withdraw body fluids, such as blood, for hemodialysis treatment. When not in use, the catheter remains in its position, commonly an intravascular position, until a subsequent treatment is performed.

The catheters that may be used accordance with this invention include known and commonly used catheters and are readily available from a variety of commercial sources. The catheters may vary in configuration and size. One type of catheter commonly used in accordance with this invention is a tunneled catheter that includes a cuff for ingrowth of tissue to anchor the catheter. Examples of catheters that may be used include, but are not restricted to, an ASH SPLIT CATH and DUOSPLIT by Ash Medical Systems (West Lafayette, Indiana) and Medcomp (Harleysville, Pennsylvania); Tesio Catheters by Medcomp; PERM CATH by Quinton Instrument Company (Seattle, Washington); and HICKMAN and VAS CATH by Bard, Inc. (Salt Lake City, Utah). Catheters containing totally subcutaneous ports are also useful in the present invention; examples include LIFESITE by Vasca (Topsfield, Maine); and DIALOCK by Biolink, Inc. of (Boston, Massachusetts).

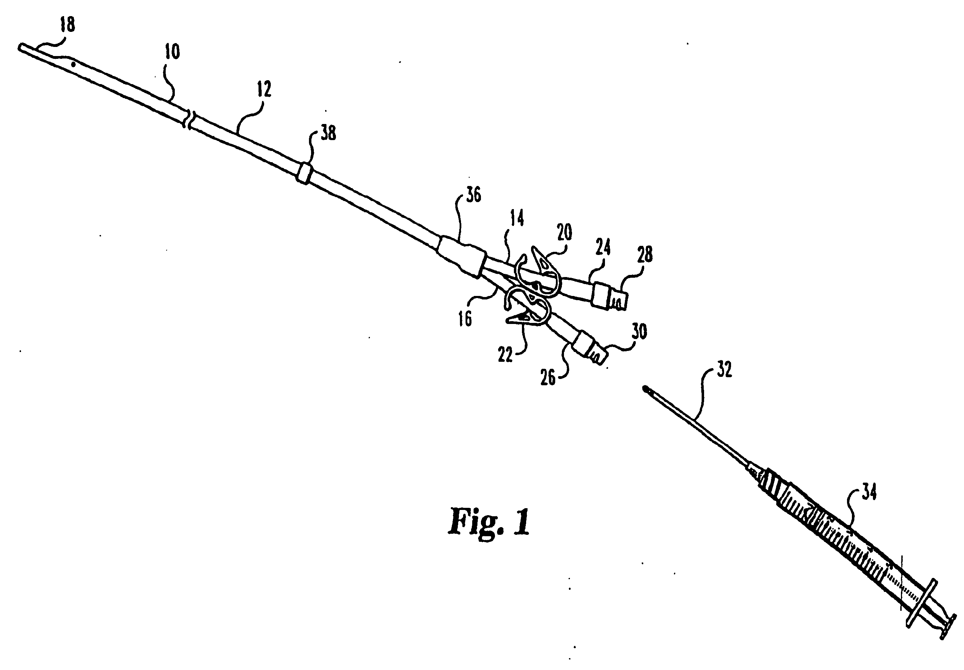

Figure 1 depicts one example of a catheter 10 for use with this invention. Catheter 10 is a dual lumen catheter and includes an outer sheath 12 having a cuff 38 and first and second lumens 14 and 16, respectively.

Lumens 14 and 16 extend from distal tip 18 through sheath 12 and exit from sheath 12 at connection 36. Each of lumens 14 and 16 include releasable clamps 20 and 22, respectively. Each of lumens 14 and 16 terminate in a threaded end 24 and 26, which can be threadedly attached to protective end caps 28 and 30, respectively. Fluids including a lock solution can be infused or withdrawn from each lumen 14 and 16 by inserting needle 32 of a syringe 34 through protective end caps 28 and/or 30 after protective end caps 28 and/or 30 have been sterilized by cleaning successively, for example with Betadine and alcohol. Alternatively, one or both protective end caps 28 and 30 can be removed and threaded ends 24 and 26 can be threadedly attached via a connector (not shown) to lines for infusion or withdrawal of fluids (not shown). Once a desired treatment session has been completed, the needles are removed or the connectors are replaced with fresh, sterile protective end caps. The lumens are then typically flushed with normal saline, after which a lock solution is injected into each lumen. All procedures are performed using standard sterile techniques well known to those skilled in the art. The catheters for use with this invention can be prepared from a variety of materials, including, for example, silicon, polyurethane, polyvinyl, silicone, or silastic elastomer.

Chronic catheters are usually inserted through an internal jugular vein into the superior vena cava. Usually these catheters include a cuff attached to the exterior of the catheter and placed under the skin, which promotes ingrowth of fibrous tissue, and thus fixes the catheter in position and prevents bacterial migration around the catheter. The catheters are manufactured to function for several months. For example, TESIO catheters can last for up to four years with proper intervention. However, in actual practice prior to the present invention, the catheters have exhibited limited longevity because of occlusion and/or infection. The catheters frequently must be removed and/or replaced upon the occurrence of occlusion and/or infection.

The present invention provides a catheter lock solution including an anticoagulant and a photo-oxidant that has an antiseptic effect in the lock solution. As used herein, the term "photo- oxidant" is intended to refer to a confound (usually an organic dye) that has photo-oxidation properties, in which the compound exhibits an increased oxidizing potential upon exposure to radiant energy such as light. The term "photo-oxidant" also refers to a composition that releases one or more electrons when struck by light. In various embodiments, the lock solution aids in the prevention of infection and occlusion of an indewelling catheter.

It is understood that the photo-oxidant should be safe for use in a catheter surgically positioned within a patient's body, such as, for example, an intravenous catheter. The photo-oxidant must also be compatible with an anticoagulant selected for use in the catheter lock solution, when present. In one preferred aspect of the invention, the photo-oxidant is methylene blue, which advantageously provides antibiotic and antifungal activity, and also provides particular advantages by reducing incidence of catheter occlusion, and providing a color to make the catheter lock solution clearly identifiable within a syringe or a catheter. In addition to methylene blue, other photo-oxidants may be used to provide antibiotic and antifungal activity. Such "other" photo-oxidants may also to provide particular by reducing incidence of catheter occlusion, and/or to provide a color to make the catheter lock solution clearly identifiable within a syringe or a catheter. For example, it is expected that alternative photo-oxidants that can be used include Rose Bengal, hypericin, methylene violet, proflavine, rivanol, acriflavine, toluide blue, trypan blue, neutral red, a variety of other dyes or mixtures thereof. Therefore, one or more alternative photo-oxidants, preferably a colored photo-oxidant is used in accordance with the invention in place of methylene blue.

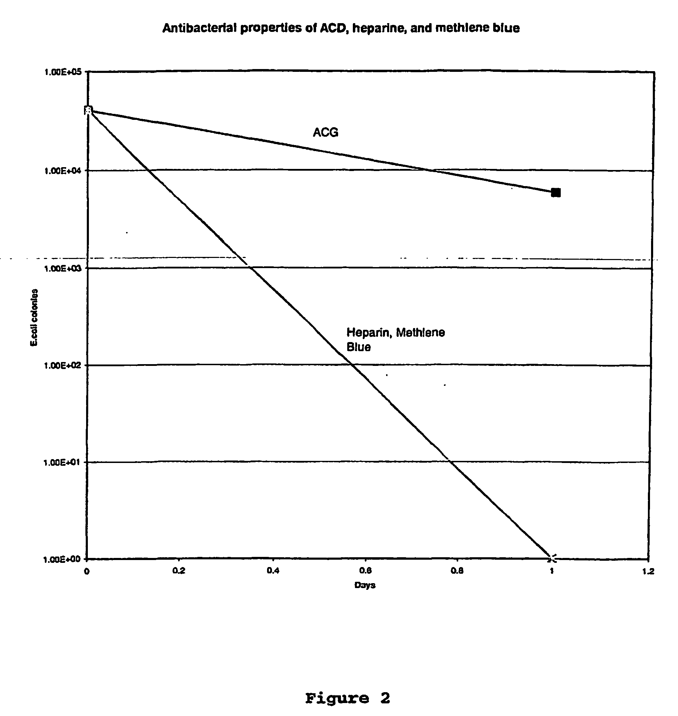

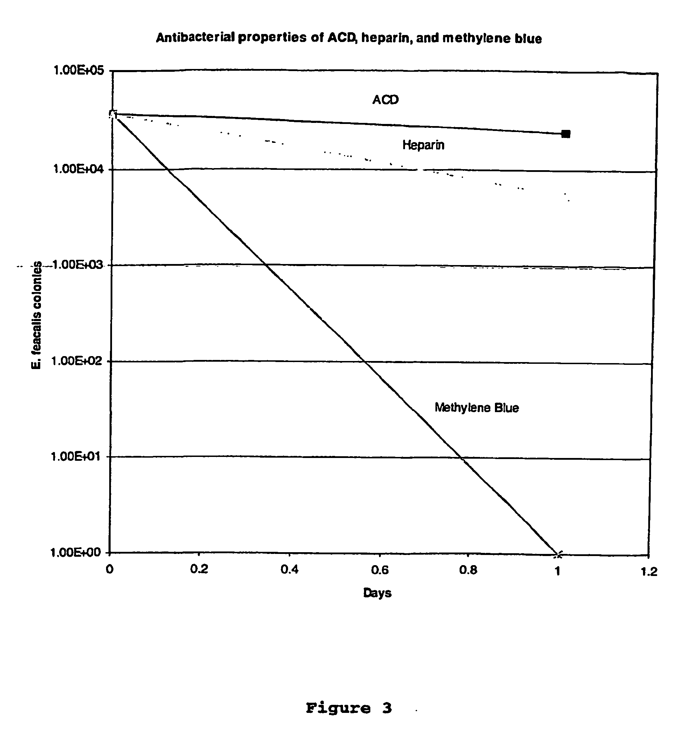

It has unexpectedly been determined that methylene blue has surprisingly effective antibacterial activity when used in accordance with the present invention, especially when exposed to light. In a series of tests, with a gram negative and a gram positive organism, methylene blue inactivated all bacteria within 1 day, while heparin and a 4% concentration of citrate caused no effect on gram positive organisms (Figures 2,3). Methylene blue also advantageously colors the lock solution blue, which provides a useful tool to medical personnel in preventing confusion between the lock solution and any other solution that might be present in a syringe or the catheter.

In vitro, methylene blue has marked bactericidal properties at relatively low concentrations. These properties do not require the presence of light, but it is expected that the bactericidal properties would be enhanced by exposure to light.

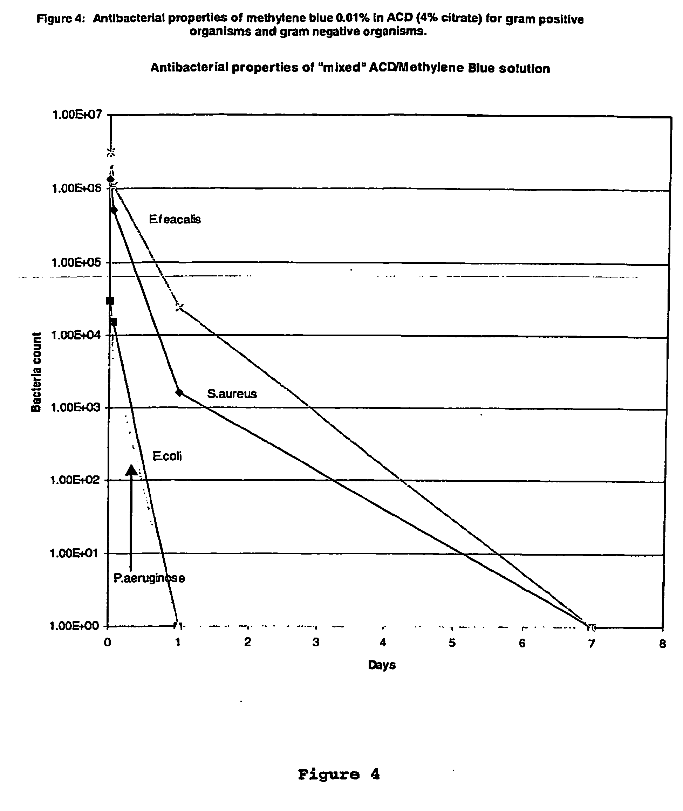

Methylene blue at a concentration of 0.01% (10 mg/100 mL) has been shown to kill gram-negative organisms within one day. In addition, methylene blue and 4% citrate at pH 4.5 (in the form of Anticoagulant Citrate Dextrose ("ACD") solution) kills gram positives such as enterococcus within one day. Figure 4 sets forth data showing the antibacterial properties of methylene blue 0.01% in ACD (4% citrate) for gram positive organisms and gram negative organisms. The data depicted in Figure 4 is also set forth below in Table I:

**Table I**

| **Organism** | **Day 0** | **Day 0.0416** | **Day 1** | **Day 7** |
|---|---|---|---|---|
| P. aeruginosa | 2.41E+06 | 4.1E+03 | 1.00E+00 | 1.00E+00 |
| E. coli | 3.00E+04 | 1.52E+04 | 1.00E+00 | 1.00E+00 |
| *S. aureus* | 1.36E+06 | 5.22E+05 | 1.60E+03 | 1.00E+00 |
| *E. faecalis* | 3.11E+06 | 1.11E+06 | 2.365E+04 | 1.00E+00 |

Although it is not intended that the present invention be limited by any theory by which it achieves its advantageous result, it is believed that the mechanism of antiseptic properties of methylene blue is through its oxidation potential. Due to the interactions of hydrogen ion with redox potential, methylene blue is expected to be more effective at a neutral pH than acidic pH.

In one form of the invention, therefore, a lock solution is provided that comprises methylene blue in a concentration effective to kill bacteria. In one embodiment, the lock solution has a methylene blue concentration of up to about 1500 mg/100 ml, preferably from about 1 mg/100 ml to about 1500 mg/100 ml. In another embodiment, the lock solution has a methylene blue concentration of from about 1 to about 1000 mg/100ml. In another embodiment, the lock solution has a methylene blue concentration of from about 1 to about 100 mg/100ml. In yet another embodiment, the lock solution has a methylene blue concentration of from about 1 to about 50 mg/100 ml. In still another embodiment, the methylene blue concentration is about 10 mg/100 ml.

Another excellent property of methylene blue when selected for use in accordance with the invention is its color, and a determination of a concentration for a given solution can be based in part upon the intensity of color that develops. The color has a safety function, indicating to observers that the catheter contains a catheter lock solution. At 10 mg/100ml, the preparation has a dark blue color in a syringe, and a noticeably blue color within the clear external segments of the catheter. Over time, the methylene blue solution lightly stains the inside of external segments made of polyurethane or silicone, but the injected lock solution still makes the segments noticeably darker in color. Therefore the presence of the lock solution is recognizable.

As stated above, a catheter lock solution in certain embodiments of the present invention also includes an anticoagulant. Examples of anticoagulants include, for example, heparin and citrate. When the anticoagulant includes heparin, the heparin is preferably present at a concentration of from about 100 units/ml to about 10,000 units/ml.

In certain preferred aspects of the invention, the anticoagulant is citrate. Citrate is preferably present at a molar concentration at least as high as the molar calcium concentration in a patient's blood. In one preferred embodiment, citrate is present in a lock solution at a concentration of from about 1.5 to about 47 percent by weight. In another embodiment, citrate is present at a concentration of from about 1.5 to about 23 percent by weight. In yet another embodiment, citrate is present at a concentration of from about 1.5 to about 15 percent by weight. In another embodiment, citrate is present at a concentration of about 7 percent by weight. In still another embodiment, citrate is present at a concentration of at least about 0.004 Molar, more preferably from about 0.01 to about 1.0 Molar. Another embodiment includes citrate at a concentration of from about 0.1 to about 0.5 Molar. Yet another embodiment includes citrate at a concentration of about 0.24 Molar.

Although it is not intended that the present invention be limited by any theory whereby it achieves its advantageous results, in a lock solution including citrate, it is believed that the citrate prevents coagulation by chelating the calcium in the adjacent blood. Thus, in one preferred embodiment, the solution contains sufficient citrate ions to effectively chelate at least an amount of calcium that would be present in a quantity of blood equivalent to the volume of a catheter lumen. In other preferred embodiments, the solution has a significantly higher citrate ion concentration. In general, the arterial and venous lumens of tunneled central venous catheters typically have an internal volume in the range of 1.5-2.5 ml. A 0.24 M citrate solution contains 0.24 mmoles/mL of citrate ion which is approximately 50 times the citrate required to chelate the calcium in the volume of blood found in a catheter lumen.

In addition, it has been found that concentrated citrate has antibacterial and antifungal properties. As stated above, information relating to the use of concentrated citrate is included in International Publication No. WO 00/10385. The ability of citrate to kill microorganisms has been shown to increase with an increase in the concentration of citrate. When citrate is mixed with large numbers of gram negative bacteria, all are killed within 1-7 days. Depending upon pH and concentration, large numbers of gram positive organisms are killed between 7 and 21 days. Fungi may also be killed by contact with citrate; however, fungi take longer to kill. It is believed that citrate's ability to kill bacteria and fungi is higher for the few numbers of organisms that contaminate the connector of a catheter during dialysis. Where a citrate concentration is selected that does not have a suitable rate of killing microorganisms, the lock solution will preferably include another antibacterial component for enhancing the antiseptic effect of the solution.

In one preferred embodiment, an inventive catheter lock solution includes 0.24 Molar citrate (provided, for example, in the form of trisodium citrate dihydrate) and methylene blue at a concentration of 10 mg/100 ml. When this solution is infused into a catheter, the methylene blue slowly penetrates through the biofilm to stain the inside of the catheter. A lock solution having this concentration of methylene blue creates a light stain of the external clamping segments of a chronic CVDC, which allows an observer to still visualize the interior to see whether it contains the lock solution, saline, or blood. As methylene blue slowly penetrates through the biofilm to stain the inside of the catheter, it is believed that citrate also penetrates through the biofilm. The presence of methylene blue and/or citrate within the biofilm and in the surface of the catheter are also believed to prevent bacterial growth in these locations.

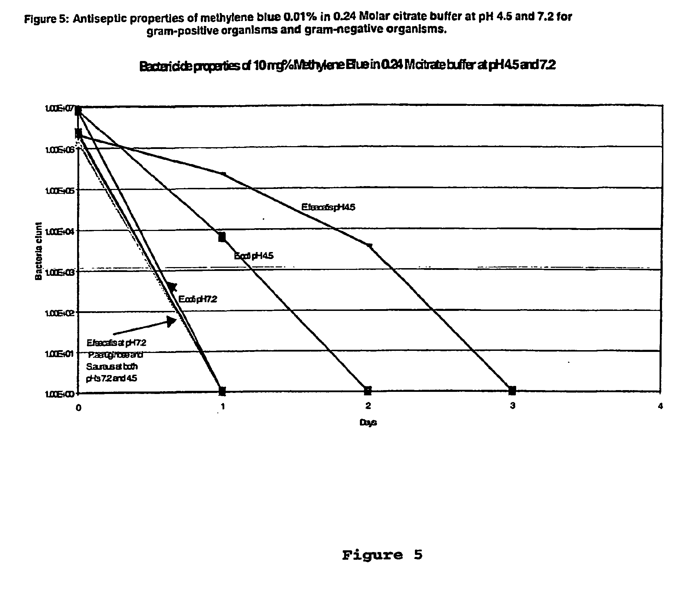

In another embodiment of the invention, a citrate/methylene blue lock solution is provided having a pH of from about 4 to about 8. In another embodiment, the solution has a pH of from about 4.5 to about 8. In still another embodiment, the solution has a pH of from about 6 to about 8. In yet another embodiment, the pH is from about 6.7 to about 7.7. Another embodiment features a pH of from about 7.0 to about 7.4. In one preferred embodiment, the pH is about 7.2. Based upon in vitro testing, and the known relationship between pH and oxidation potential, it is believed that antimicrobial effects of methylene blue are greater at approximately neutral pH than at acidic pH. For example, citrate at 7% concentration in the presence of methylene blue at a concentration of 10 mg/100 ml has been found to be more effective at approximately neutral pH than at 4.5 pH. Specifically, an inventive citrate/methylene blue catheter lock solution including 0.24 Molar citrate (as trisodium citrate dihydrate) and 10 mg/100 ml methylene blue at pH 7.2 ± 0.1 pH units and with a relative density of from about 1.035 to about 1.045 at 20°C has been tested to determine the antibacterial properties, against both gram-negative and gram-positive organisms. The results, which are set forth in Figure 5, indicate highly effective antibacterial properties. The data represented in Figure 5 are also set forth below in Table II:

**Table II**

| | ***E. coli*** | | ***P. aeruginosa*** | | ***S. aureus*** | | ***E. faecalis*** | |
|---|---|---|---|---|---|---|---|---|
| **DAY** | **pH 7.2** | **pH 4.5** | **pH 7.2** | **PH 4.5** | **pH 7.2** | **PH 4.5** | **pH 7.2** | **pH 4.5** |
| **0** | 3.15E+06 | 8.15E+06 | 1.45E+06 | 1.45E+0.6 | 2.36E+06 | 2.36E+06 | 2.1E+06 | 2.1E+06 |
| **1** | 1.00E+00 | 6.5E+03 | 1.00E+00 | 1.00E+00 | 1.00E+00 | 1.00E+00 | 1.00E+00 | 2.34E+05 |
| **2** | 1.00E+00 | 1.00E+00 | 1.00E+00 | 1.00E+00 | 1.00E+00 | 1.00E+00 | 1.00E+00 | 3.9E+03 |
| **3** | 1.00E+00 | 1.00E+00 | 1.00E+00 | 1.00E+00 | 1.00E+00 | 1.00E+00 | 1.00E+00 | 1.00E+00 |

A problem with all catheter lock solutions is that they do not permanently stay within the catheter. Some of the catheter lock solution exits the end of the catheter during the infusion (often about 1/3 of the injected volume). In addition, the portion remaining in the end of the catheter is typically washed out slowly by flow of blood through the side-holes of the catheter (if present). Other lock solution slowly diffuses from the body of the catheter through the end of the catheter during the time that lapses between dialysis treatments.

In the case of concentrated citrate, for example, gravitational effects also come into play. It is of course understood that the densities of citrate solutions increase as the concentrations of citrate therein increase. The relative density of 23% citrate, for example, is 1.120, which is significantly higher than the relative density of blood. Thus, when the patient is erect, the segment of the inner portion of the catheter in the vena cava is vertical. Gravitational force causes citrate at this concentration to slowly leave the catheter. In the laboratory, in some types of catheters positioned vertically (such as the double-D shaped Ash Split Cath catheters), 23% citrate lock can be shown to slowly exit from the distal part of the catheter over 3-5 days, into blood or blood substitute (with the same relative density). In other catheters (such as cylindrical Tesio catheters) the 23% citrate lock does not exit over time.

From bacteriologic studies and initial clinical trial results, it is believed that the optimal concentration of citrate in a catheter lock solution in the absence of other components having significant antibacterial effect is at least 10% concentration, with a pH of 4.5. However, for this concentration of citrate to be effective, it should remain within the catheter lumen between dialysis treatments. In vitro studies have indicated that the density of the lock solution is critically important in determining the length of time that the lock solution remains in the catheter. The relative density of blood with hematocrit of 32% is approximately 1.045. If a catheter lock solution with relative density higher than this is placed into a catheter positioned vertically, the lock solution will exit from the catheter at a slow rate. Increasing the viscosity with polymeric substances such as PEG slows but does not prevent the egress of the lock solution. Therefore, in certain embodiments of the invention, the citrate concentration in a lock solution is selected such that the density of the lock solution is sufficiently close to the density of the patient's blood that the solution does not exit the catheter during the lock period to an unacceptable degree. It is believed that 0.24 Molar citrate alone does not have significant antibacterial effect at neutral or acid pH; however, the antithrombotic effect of 0.24 Molar citrate will remain very high even with some diffusion out of the catheter, and the antibacterial function in the lock solution can be supplemented in accordance with the invention, for example, by including methylene blue in the solution.

In one embodiment of the invention, therefore, the catheter lock solution has a density of from about 1.000 to about 1.300 g/ml. In another embodiment, the lock solution has a density of from about 1.000 to about 1.080 g/ml. In still another embodiment, the lock solution has a density of from about 1.030 to about 1. 050 g/ml. In yet another embodiment, the lock solution has a density of from about 1.035 to about 1.045 g/ml. It is understood that the density of a given patient's blood may differ from the density of the blood of another patient; however, matching the relative density of the catheter lock solution to the relative density of whole blood of a patient is well within the purview of a person of ordinary skill in the art. Closely matching the densities has the advantageous effect of aiding in the retention of the catheter lock solution within the catheter between treatments. When the relative densities are relatively close, gravitational force does not tend to urge the catheter lock solution out of the catheter when the patient is upright. Similarly blood will not enter the catheter when the catheter is upward directed as in the femoral vein and the patient is standing (as can happen with a low-density catheter lock such as heparin).

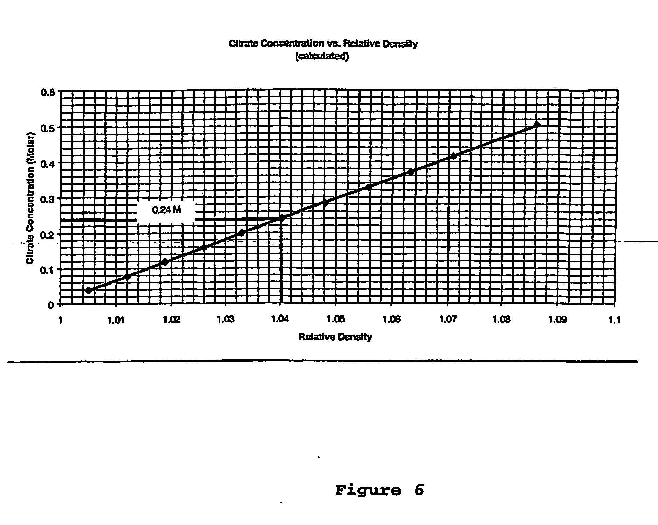

The densities of various formulations of various catheter lock solutions are directly related to the citrate concentration, as reflected in the linear graph set forth in Figure 6. In a preferred embodiment, the density of the lock solution is less than blood with a hemocrit of 32 (i.e., from about 1.035 to about 1.045). As such, in one embodiment, the lock solution has a citrate concentration of up to about 0.24M (i.e., up to about 7% by weight). A solution having a citrate concentration of 0.24M has a relative density of about 1.045. This concentration of citrate does not have strong antiseptic properties, but is still highly effective as an anticoagulant even if diluted by blood at the tip of the catheter. The antiseptic function can be supplemented in accordance with the invention by including methylene blue in the lock solution with the citrate. Indeed, a citrate concentration of about 7% provides anticoagulant properties superior to concentrated heparin. A higher concentration of citrate would be desirable for antiseptic and anticoagulant effect; however, in order to retain a significant proportion of the citrate within the catheter body, a density that is more closely aligned with blood density is preferred.

In another aspect of the invention, the catheter lock solution may also include an agent to increase viscosity, as described in International Publication No. WO 00/10385. The presence of a viscosifying agent is particularly useful, for example, when the relative density of a given catheter lock solution is not the same as the density of a patient's blood.

Therefore, in certain preferred embodiments, a lock solution is provided that comprises a photo-oxidant, an anticoagulant and one or more agents to adjust viscosity to help retain the lock within the catheter for a desired amount of time. It is well known that catheters are manufactured to have a variety of configurations and lumen diameters. For example, catheters can include single or double lumens. The double lumens can be fused adjacent to each other or they can be concentric. The lumens can have varying cross-sectional areas and shapes, ranging from substantially circular to substantially ovoid. As discussed above, a phenomenon common to most lock solutions is that a portion of the solution at the distal end of the lumen diffuses into the patient's blood stream and is replaced in the catheter by blood. The rate of diffusion of a lock solution from a lumen can be influenced not only by the density of the lock solution, but also by the cross-sectional shape and area of the particular lumen(s) and the viscosity of the lock solution. A lock solution of the present invention is preferably prepared to have a viscosity and density such that a substantial portion of the lock solution does not diffuse or flow out of a catheter lumen within several days.

Viscosifying agents that can advantageously be selected for use in accordance with the present invention include those pharmaceutically acceptable agents known or commonly used in treatment of animals including humans. Examples include, but are not limited to, dextran, polyethylene glycol, glycerin, polygeline, and non-metabolizable sugars such as sorbitol and mannitol and mixtures of these compounds. An excellent aspect of the invention, therefore is a composition useful as a lock solution that comprises methylene blue (or other photo-oxidant), a citrate salt (or other anticoagulant) and a viscosifying agent. The viscosifying agent allows a higher concentration of citrate to be used without having an unacceptable degree of egress of the lock solution from the catheter due to high density of the lock solution.

While it is understood that optimal viscosity and density are dependent upon the shape and size of a particular lumen, a person of ordinary skill in the art, in view of the description herein, can readily determine a desired density and viscosity for a particular catheter without undue experimentation. It is of course understood that the need for viscosifying agents is reduced in a lock solution having a relatively lower concentration of citrate and a density closely matched to that of blood. The antiseptic effect of the citrate, which is reduced by the reduction in the citrate concentration, is replaced by the inclusion of methylene blue or other photo-oxidant in an amount that provides an antiseptic effect.

An inventive lock solution can be prepared to include a variety of other pharmaceutically acceptable agents. For example, the lock solution can include salts, such as, for example, sodium chloride or other sodium salts. The lock solution can also include a variety of other antibacterial, antimicrobial and anticoagulant agents. Such antibacterial and antimicrobial agents are well known to those skilled in the art and can include, without limitation, gentamicin, vancomycin, and mixtures of these agents. Additional anticoagulant agents include, for example, urokinase, tissue plasminogen activation (tPA)and mixtures of these agents.

By "pharmaceutically acceptable", it is meant that the lock solution and the included salts and other additives which are, within the scope of sound medical judgment, suitable for use in contact with tissues of humans and lower animals without undue toxicity, irritation, allergic response, and the like, and are commensurate with the reasonable benefit/risk ratio. It is also typically necessary that a composition be sterilized to reduce the risk of infection. For example, pharmaceutically acceptable salts are well-known in the art, for example, as found in S.M. Berge et al. described in detail in J. pharmaceutical Science, 66:1-19, 1977.

Once a lock solution in accordance with the invention is infused into the lumen of a catheter, it is preferably allowed to remain until that particular catheter or lumen is desired to be accessed again. Especially with heparin, it is important to remove the catheter lock before starting the dialysis procedure, or using the catheter for fluid infusion.

A great advantage of methylene blue or other colored photo-oxidant selected in accordance with the invention, as discussed above, is that it provides color to the lock solution. This color indicates to health professionals using the catheter that it is filled with a catheter lock solution. Indeed, in accordance with another aspect of the invention, different colorants are included in various solutions used in connection with catheters and/or syringes in a given system so that medical personnel will be able to ascertain what type of solution is in the catheter or syringe by simply observing the color of the solution. In this regard, a plurality of photo-oxidant compositions in accordance with the invention may be used in a color-coding system.

In another excellent feature of the invention, a lock solution including methylene blue or other photo-oxidant is exposed to light either before or after infusion into an indwelling catheter. While it is not intended that the invention be limited by any theory whereby it achieves its advantageous result, it is believed that the antibacterial effect of the methylene blue and other photo-oxidants is enhanced by exposure to light. The exposure can be achieved, for example, by exposing the solution to ambient light prior to infusion into the catheter, by exposing the solution to high intensity light prior to infusion into the catheter, or by exposing the solution to one or more pulses of light after it is infused into the catheter. The present invention also contemplates the placement of a light source on or near an indwelling catheter at various locations to provide a manner in which to expose a lock solution to light as it resides in the catheter.

The catheter lock solution containing methylene blue or other photo-oxidant may be injected into catheters used for access to other body spaces besides veins or arteries. For example, catheters used in peritoneal dialysis access the peritoneum (the space defined by the peritoneal membrane and exterior to the organs in the abdomen). These catheters also have a risk of bacterial and fungal contamination. After draining and infusing peritoneal dialysate solutions, a lock solution including methylene blue or other photo-oxidant is infused into the catheter. Other catheters with risk of infection include catheters in the urinary bladder, the cerebral spinal fluid (around the central nervous system) and the subcutaneous space (under the skin).

In another aspect of the invention, there is provided a catheter lock kit. In one preferred embodiment, a kit includes four sterile components as follows; (1) 5cc of catheter lock solution (such as the solution described in Example 3); (2) a 3cc syringe with Luer lock tip; (3) a replacement cap; and (4) a needleless single-dose vial access spike with dead cap. The catheter lock solution can be advantageously provided in the form of a 5 mL vial that has been aseptically filled with the solution. A suitable sterile single use hypodermic syringe is commercially available from Becton Dickinson, One Becton Drive, Franklin Lakes, NJ. A suitable catheter lumen replacement cap is commercially available from B. Braun Medical, Inc., 824 12^{th} Avenue, Bethlehem, PA. A suitable single dose vial access spike is commercially available from ICU Medical, Inc., 951 Calle Amanecer, San Clemente, CA.

As can be appreciated by those of skill in the art, in one embodiment there has been described an aqueous catheter lock fluid comprising an anticoagulant and a photo-oxidant, wherein the fluid has a density of from about 1.000 to about 1.300 g/ml. In certain embodiments, the photo-oxidant comprises a member selected from the group consisting of methylene blue, Rose Bengal, hypericin, methylene violet, proflavine, rivanol, acriflavine, toluide blue, trypan blue, neutral red and mixtures thereof. The photo-oxidant has an antiseptic effect. In certain preferred embodiments, the photo-oxidant comprises methylene blue. The concentration of methylene blue in the fluid is preferably up to about 1500 mg/100 ml.

In certain embodiments, the anticoagulant comprises a member selected from the group consisting of citrate, heparin, urokinase, tissue plasminogen activation (tPA) and mixtures thereof. In one preferred embodiment, the anticoagulant comprises citrate. In another embodiment, the concentration of citrate in the fluid is at least as high as the calcium concentration in a patient's blood. In still another form of the invention, the concentration of citrate in the fluid is from about 1.5 to about 47% by weight. In yet another embodiment, the concentration of citrate in the fluid is from about 0.01 to about 1.0 Molar.

In one preferred embodiment, the fluid comprises citrate and methylene blue. In another embodiment, the concentration of citrate in the fluid is from about 1.5 to about 23% by weight and the concentration of methylene blue in the fluid is from about 1 to about 1000 mg/100 ml. In a preferred embodiment, the concentration of citrate in the fluid is about 7% by weight and the concentration of methylene blue in the fluid is about 10 mg/100 ml. In one embodiment, the pH of the fluid is from about 4 to about 8. In another embodiment, the pH of the fluid is from about 6 to about 8. In another embodiment, the pH of the fluid in about 7.2. In another embodiment, the relative density of the fluid is from about 1.000 to about 1.080 g/ml. In another embodiment, the relative density of the fluid is from about 1.035 to about 1.045 g/ml.

In yet another embodiment, the fluid further comprises a viscosifying agent. The viscosifying agent can be, for example, a member selected from the group consisting of dextran, polyethylene glycol, glycerin, polygeline, and non-metabolizable sugars such as sorbitol and mannitol and mixtures of these compounds.

In another embodiment, the photo-oxidant features a readily detectible color, thereby allowing healthcare professionals to readily recognize the fluid, avoiding accidental infusion of the fluid into a patient's bloodstream.

In another form of the invention, there is provided an infusion device for infusing a lock solution into a lumen of a catheter. The device includes: (1) a syringe; and (2) a pharmaceutically acceptable lock solution in accordance with the invention contained within the syringe; wherein the syringe containing the lock solution is sterilized.

Also provided is a kit for locking a patient's catheter. The kit includes: (1) a container having therein an inventive catheter lock solution; (2) a syringe with Luer lock tip; (3) a replacement cap; and (4) a needleless single-dose vial access spike with dead cap.

The invention will be further described with reference to the following specific Examples. It will be understood that these Examples are also illustrative and not restrictive in nature.

### EXAMPLE 1

FIG. 2 is a representation of the effect of methylene blue (12 mg/100ml) on concentration of a gram negative organism, Escherichia Coli, without light exposure. All E. Coli are killed within one day. It is expected that illumination will augment bacterial killing by methylene blue even more.

### EXAMPLE 2

Fig 3 is a representation of the effect of methylene blue (12 my/100ml) on concentration of a gram positive organism, Enterobacter Faecalis, without light exposure. All Enterobacter are killed within one day. It is expected that illumination will augment bacterial killing by methylene blue even more.

### EXAMPLE 3

### Manufacturing of a Representative Catheter Lock Solution

### Method

A catheter lock solution is formulated as a sterile mixture of USP grade chemicals in the following concentrations: 0.24 M citrate buffer solution and 0.01% (W/V) methylene blue. The solution is designed to have a relative density of 1.035 to 1.045, and pH of 7.1-7.3. The citrate buffer solution is prepared at the desired pH (7.1-7.3) by mixing one liter of 0.24 M trisodium citrate dihydrate solution (70.58g/L) and 6.5 ml of 0.24 M anhydrous citric acid solution (46.10 g/L). The final solution is obtained by adding 0.0117 grams (11.7 mg) of methylene blue trihydrate per 100 ml of citrate buffer solution in the actual batch size. The solution is stored at room temperature: however, brief exposure up to 50° C (122° F) does not adversely affect the product.

The bulk solution is then pumped into an aseptic filling area, passing through a secondary and then primary 0.2 micron sterilizing filter before flowing into a sterilized surge type or pressure type vessel. The sterilized solution in the sterile vessel flows to the filler where light resistant, type 1 glass vials (5 mL, Kimble, type 1 Borosilicate Glass Amber Vial, 13-mm Finish, Untreated)are conveyed and filled with the predetermined fill volume. The filled vials are then conveyed to the stoppering location where stoppers (West, 13 mm, 4432/50 Rubber Stopper) are placed in the vials. The vials are then conveyed to a capping machine which applies aluminum crimp seals with flip off caps to each vial (West, 13 mm Aluminum Seal, Flip-off Button). Overseals (crisped caps) are applied in a capping area outside of the aseptic processing area.

The filled, stoppered and capped vials are then inspected for visible particulate matter and other defects.

### Suppliers

The starting materials for making the solution of this embodiment are readily available commercially, and can be obtained from the sources identified in Table III:

**Table III**

| Chemical Name | Supplier | Concentration |
|---|---|---|
| Citric Acid Anhydrous, USP | Spectrum Lab Product 14422 Sooth San Pedro St. Gardena, CA 90248 | USP |
| Sodium Citrate, Tri basic Dihydrate, USP | Sigma-Adrich 3050 Spruce St. St. Louis, MO 63103 | USP |
| Methylene Blue, USP | Spectrum Lab Product 14422 South San Pedro St. Gardena, CA 90248 | USP |
| WFI, USP | Contract Mfg | USP |

### EXAMPLE 4

The antimicrobial effectiveness after one day and three days of the citrate/methylene blue catheter lock solution described in Example 3 was tested under USP procedures (10 mL sample). Results are shown in Table IV below:

**TABLE IV**

| **Organism** | **Day Zero** | **Day 1** | **Day 3** |
|---|---|---|---|
| *Escherichia coli* | 1.1 x 10⁵ | 67 x 10³ | 1.9 x 10⁴ |
| *Pseudomonas aeruginosa* | 1.5 x 10⁵ | 1.9 x 10³ | 4.1 x 10⁴ |
| *Candida albicans* | 1.6 x 10⁵ | 3.8 x 10⁵ | 3.6 x 10⁵ |
| *Staphylococcus aureus* | 8.3 x 10² | 5.0 x 10⁰ | <1.0 x 10⁰ |
| *Aspergillus niger* | 2.5 x 10⁵ | 3.4 x 10⁵ | 3.7 x 10⁵ |

The conclusion of this test is that the solution achieved a 1.0 log kill for bacteria and no change for fungi at one (1) day. Staphylococcus Aureus experienced an immediate 3 log kill upon inoculation.

### Example 5

As part of a shelf life study, antimicrobial effectiveness of the solution described in Example 3 was tested after one day and three days under USP procedures (2 mL sample) using three bacteria and one fungus. This study was conducted 45 days after production. The results of this study are shown in Table V below:

**TABLE V**

| **Organism** | **Day Zero** | **Day 1** | **Day 3** |
|---|---|---|---|
| *Escherichia coli* | 1.4 x 10⁵ | 2.9 x 10³ | 10 x 10² |
| *Pseudomon*as *aeruginosa* | 4.1 x 10⁵ | 3.0 x 10² | 2.5 x 10⁵ |
| *Candida albicans* | 1.4 x 10⁶ | 6.8 x 10⁴ | 2.5 x 10⁴ |
| *staphylococcus aureus* | 7.5 x 10⁴ | <1.0 | <1.0 |

The conclusion is that there was no decline in antimicrobial effectiveness of the solution following. 45 days of storage at room temperature.

### EXAMPLE 6

The catheter lock solution is removed before each dialysis procedure, by attaching a syringe to each catheter lumen and removing 1 mL more than the catheter lumen volume (about 3 mL total), discarding the syringe, then flushing the catheter with 5 mL of sterile normal saline.

At the end of the patient's hemodialysis treatment each lumen of the catheter is filled with the lock solution in an amount equal to the fill volume of the catheter lumen. Each lumen is filled to the tip using a quick bolus technique for the first 2/3 of the injected volume, and slow infusion (over 10 seconds) for the last 1/3 of the injected volume.

## Claims

1. An aqueous catheter lock fluid comprising citrate as an anticoagulant and a photo-oxidant, wherein the photo-oxidant has an antiseptic effect.

2. The fluid according to claim 1 wherein the photo-oxidant comprises a member selected from the group consisting of methylene blue, Rose Bengal, hypericin, methylene violet, proflavine, rivanol, acriflavine, toluide blue, trypan blue, neutral red and mixtures thereof

3. The fluid according to any of claims 1 or 2 wherein the fluid has a density of from about 1.000 to about 1.300 g/ml.

4. The fluid according to claim 1 wherein the photo-oxidant comprises methylene blue.

5. The fluid according to claim 4 wherein the concentration of methylene blue in the fluid is up to 1500 mg/100 ml.

6. The fluid according to claim 4 wherein the concentration of methylene blue in the fluid is from 1 to 100 mg/100 ml.

7. The fluid according to claim 1 or 2 wherein the concentration of citrate in the fluid is at least as high as the calcium concentration in a patient's blood.

8. The fluid according to claim 1 or 2 wherein the concentration of citrate in the fluid is from 1,5 to 47% by weight.

9. The fluid according to claim 1 or 2 wherein the concentration of citrate in the fluid is from 0.01 to 1.0 Molar.

10. The fluid according to claim 1 wherein the fluid comprises citrate and methylene blue.

11. The fluid according to claim 10 wherein the concentration of citrate in the fluid is from about 1.5 to about 23% by weight and wherein the concentration of methylene blue in the fluid is from 1 to 1000 mg/100 ml.

12. The fluid according to any of claims 1 or 2 wherein the pH of the fluid is from 6 to 8.

13. The fluid according to any of claims 1 or 2 wherein the pH of the fluid is from 7.0 to 7.4.

14. The fluid according to any of claims 1 or 2, further comprising a viscosifying agent.

15. The fluid according to any of claims 1 or 2, wherein the photo-oxidant features a readily detectible color, thereby allowing healthcare professionals to readily recognize the fluid, avoiding accidental infusion of the fluid into a patient's bloodstream.

16. The use of an anticoagulant and a photo-oxidant, wherein the photo-oxidant has an antiseptic effect, for the manufacture of a medicament in the form of a catheter lock solution for infusion into the lumen of a catheter for preventing passage of infection along the catheter.

17. The use of claim 16 wherein the lock solution is for in a catheter selected from the group consisting of an intravascular catheter and a body cavity catheter.

18. The use of claim 16 wherein the lock solution is for infusion in the catheter in an amount of from 80% to 120% of the internal volume of the lumen of the catheter.

19. The use of claim 16 wherein the solution has a density from 1.000 to 1.300 g/ml.

20. An infusion device for infusing a lock solution into a lumen of a catheter, said device comprising:
a syringe;
a pharmaceutically acceptable lock solution contained within the syringe, said lock solution including citrate as an anticoagulant and a photo-oxidant wherein the photo-oxidant has an antiseptic effect and said syringe containing the lock solution is sterilized.

21. The device of claim 20, wherein the photo-oxidant comprises methylene blue.

22. The device of any of claims 20 or 21 wherein the lock solution comprises a viscosifying agent.

23. A kit for locking a patient's catheter, comprising:
a container having therein a catheter lock solution, the catheter lock solution comprising citrate as an anticoagulant and a hoto-oxidant, wherein the photo-oxidant has an antiseptic effect;
a syringe with Luer lock tip;
a replacement cap; and
a needleless single-dose vial access spike with dead cap.

24. The kit according to claim 23 wherein the photo-oxidant is methylene blue.

## Patentansprüche

1. Wäßrige Katherersperrflüssigkeit mit einem Citrat als Antikoagulans und einem Photooxidationsmittel, wobei das Photooxidationsmittel eine antiseptische Wirkung hat,

2. Flüssigkeit nach Anspruch 1, wobei das Photooxidationsmittel einen Bestandteil aufweist, der aus der Gruppe ausgewählt ist, die aus Methylenblau, Diodeosin, Hypericin, Methylenviolett, Proflavin, Rivanol, Acriflavin, Toluidblau, Trypanblau, Neutralrot und deren Gemischen besteht.

3. Flüssigkeit nach Anspruch 1 oder 2, wobei die Flüssigkeit eine Dichte von etwa 1,000 bis etwa 1,300 g/ml aufweist.

4. Flüssigkeit nach Anspruch 1, wobei das Photooxidationsmittel Methylenblau aufweist.

5. Flüssigkeit nach Anspruch 4, wobei die Konzentration von Methylenblau in der Flüssigkeit bis zu 1500 mg/100 ml beträgt.

6. Flüssigkeit nach Anspruch 4, wobei die Konzentration von Methylen blau in der Flüssigkeit 1 bis 100 mg/100 ml beträgt.

7. Flüssigkeit nach Anspruch 1 oder 2, wobei die Citratkonzentration in der Flüssigkeit mindestens so hoch wie die Calciusnkonzentravion im Blut eines Patienten ist.

8. Flüssigkeit nach Anspruch 1 oder 2, wobei die Citratkonzentration in der Flüssigkeit 1,5 bis 47 Gew.-% beträgt.

9. Flüssigkeit nach Anspruch 1 oder 2, wobei die Citratkonzentration in der Flüssigkeit 0.01- bis 1,0-molar ist,

10. Flüssigkeit nach Anspruch 1, wobei die Flüssigkeit Citrat und Methylenblau aufweist

11. Flüssigkeit nach Anspruch 10, wobei die Citratkonzentration in der Flüssigkeit etwa 1,5 bis etwa 23 Gew.-% beträgt, und wobei die Methylenblaukonzentration in der Flüssigkeit 1 bis 1000 mg/100 ml beträgt.

12. Flüssigkeit nach einem der Ansprüche 1 oder 2, wobei der pH-Wert der Flüssigkeit 6 bis 8 beträgt.

13. Flüssigkeit nach einem der Ansprache 1 oder 2, wobei der pH-Wert der Flüssigkeit 7,0 bis 7,4 beträgt.

14. Flüssigkeit nach einem der Ansprüche 1 oder 2, die ferner ein viskositätserhöhende Mittel aufweist.

15. Flüssigkeit nach einem der Ansprüche 1 oder 2, wobei das Photooxidationsmittel eine leicht nachweisbare Farbe aufweist, wodurch die Flüssigkeit für Fachleute in der Gesundheitsfürsorge leicht erkennbar ist und eine unbeabsichtigte Infusion der Flüssigkeit in den Blutkreislauf eines Patienten vermieden wird.

16. Verwendung eines Antikoagulans und eines Photooxidationsmttels, wobei das Photooxidationsmittel eine antiseptische Wirkung hat, für die Herstellung eines Medikaments in Form einer Kathetcrsperrflüssigkeit zur Infusion in das Limen eines Katheten, um die Passage einer Infektion entlang dem Katheter zu verhindern.

17. Verwendung nach Anspruch 16, wobei die Sperrflüssigkeit in einem Katheter verwendbar ist, dur aus der Gruppe ausgewählt ist, die aus einem intravaskulären Katheter und einem Körperhöhlenkatheter besteht.

18. Verwendung nach Anspruch 16, wobei die sperrflüssigkeit zur Infusion in den Katheter in einem Anteil von 80% bis 120% des Innenvolumens der Kathetcrlumens dient.

19. Verwendung nach Anspruch 16, wobei die Lösung eine Dichte von 1,000 bis 1,300 g/ml hat.

20. Infusionsgerät zur Infusion einer Sperrflüssigkeit in ein Lumen eines Katheters, wobei das Gerät aufweist:
eine Injektionsspritze;
eine in der Injektionsspritze enthaltene pharmazeutisch akzeptierbare Sperrflüssigkeit, wobei die Sperrflüssigkeit Citrat als Antikoagulans und ein Photooxidationsmittel enthält, wobei das Photooxidationsmittel eine antiseptische Wirkung hat und die Injektionsspritze, welche die Sperrflüssigkeit enthält, sterilisiert ist.

21. Gerät nach Anspruch 20, wobei das Photooxidationsmittel Methylenblau aufweist.

22. Gerät nach einem der Ansprüche 20 oder 21, wobei die Sperrflüssigkeit ein viskositätserhöhendes Mittel aufweist.

23. Besteck zum Sperren des Katheters eines Patienten, das aufweist:
einen Behälter mit einer darin enthaltenen Kathetersperrflüssigkeit, wobei die Kathetersperrflüssigkeit Citrat als Antikoagulans und ein Photooxidationsmittel aufweist, wobei das Photooxidationsmittel eine antiseptische Wirkung hat;
eine Injektionsspritze mit Luer-Kanüle;
eine Austauschkappe; und
einen nadellosen Aufstechdorn mit Blindkappe für Einzeldosisampullen.

24. Besteck nach Anspruch 23, wobei das Photooxidationsmittel Methylenblau ist.

## Revendications

1. Fluide de verrouillage de cathéter aqueux comprenant un citrate en tant qu'anti-coagulant et un photo-oxydant, dans lequel te photo-oxydant présente un effet anti-septique.

2. Fluide suivant la revendication 1, dans lequel le photo-oxydant comprend un élément choisi dans le groupe constitué de bleu de méthylène, de rose Bengale, d'hypéricine, de violet de méthylène, de proflavine, de rivanol, d'acriflavine, de bleu de toluide, de bleu de trypan, de rouge neutre et de mélanges de ceux-ci.

3. Fluide suivant l'une quelconque des revendications 1 et 2, dans lequel le fluide possède une densité d'environ 1.000 à environ 1,300 g/ml.

4. Fluide suivant la revendication 1, dans lequel le photo-oxydant comprend du bleu de méthylène.

5. Fluide suivant la revendication 4, dans lequel la concentration du bleu de méthylène dans le fluide est jusqu'à 1.500 mg/100 ml.

6. Fluide suivant la revendication 4, dans lequel la concentration du bleu de méthylène dans le fluide est de 1 à 100 mg/100 ml.

7. Fluide suivant la revendication 1 au 2, dans lequel la concentration de citrate dans le fluide est au moins aussi élevée que la concentration de calcium dans le sang du patient.

8. Fluide suivant la revendication 1 ou 2, dans lequel la concentration de citrate dans le fluide est de 1,5 à 47% en poids.

9. Fluide suivant la revendication 1 ou 2, dans lequel la concentration de citrate dans le fluide est de 0,01 à 1,0 molaire.

10. Fluide suivant la revendication 1, où le fluide comprend un citrate et du bleu de méthylène.

11. Fluide suivant la revendication 10, dans lequel la concentration de citrate dans le fluide est d'environ 1,5 à environ 23 % en poids et dans lequel la concentration du bleu de méthylène dans le fluide est d'environ 1 à 1.000 mg/100 ml.

12. Fluide suivant l'une quelconque des revendications 1 et 2, dans lequel le pH du fluide est de 6 à 8.

13. Fluide suivant l'une quelconque des revendications 1 et 2, dans lequel le pH du fluide est de 7,0 à 7,4.

14. Fluide suivant l'une quelconque des revendications 1 et 2, comprenant en outre un agent augmentant la viscosité.

15. Fluide suivant l'une quelconque des revendications 1 et 2, dans lequel le photo-oxydant comporte une couleur facilement détectable, permettant ainsi à des professionnels de la santé de reconnaître facilement le fluide, éventant une perfusion accidentelle du fluide dans la circulation sanguine d'un patient.

16. Utilisation d'un anti-coagulant et d'un photo-oxydant, dans laquelle le plioto-oxydant présente un effet anti-septique, pour la fabrication d'un médicament sons la forme d'une solution de verrouillage de cathéter pour une perfusion dans le lumen d'un cathéter pour prévenir le passage d'une infection le long du cathéter.

17. Utilisation suivant la revendication 16, dans laquelle la solution de verrouillage est à utiliser dans un cathéter choisi à partir du groupe constitué d'un cathéter intra-vasculaire et d'un cathéter de cavité corporelle.

18. Utilisation suivant la revendication 16, dans laquelle la solution de verrouillage est pour une perfusion dans le cathéter dans une quantité de 80% à 120% du volume interne du lumen du cathéter

19. Utilisation suivant la revendication 16, dans laquelle la solution possède une densité de 1,000 à 1,300 g/ml.

20. Dispositif de perfusion pour perfuser une solution de verrouillage dans un lumen d'un cathéter, ledit dispositif comprenant:
une seringue;
une solution de verrouillage pharmaceutiquement acceptable contenue dans la seringue, ladite solution de verrouillage incluant un citrate en tant qu'anti-coagulant et un photo-oxydant, où le photo-oxydant présente un effet anti-septique et ladite seringue contenant la solution de verrouillage est stérilisée.

21. Dispositif suivant la revendication 20, dans lequel le photo-oxydant comprend du bleu de méthylène.

22. Dispositif suivant l'une quelconque de la revendication 20 ou 21, dans lequel la solution de verrouillage comprend un agent augmentant la viscosité.

23. Kit pour le verrouillage d'un cathéter d'un patient, comprenant:
un récipient avec à l'intérieur une solution de verrouillage de cathéter, la solution de verrouillage de cathéter comprenant un citrate en tant qu'anti-coagulant et un photo-oxydant, où le photo-oxydant présente un effet anti-septique;
une seringue avec un embout à verrouillage Luer;
un bouchon de remplacement: et
une pointe d'accès à un flacon à dose unique sans aiguille avec un bouchon sans issue.

24. Kit suivant la revendication 23, dans lequel le photo-oxydant est du bleu de méthylène.
